# EUROPEAN PATENT APPLICATION

(11) **EP 4 374 693 A1**
(43) Date of publication of application: **29.05.2024**
(21) Application number: 22846288.3
(22) Date of filing: 22.07.2022
(51) Int. Cl.: A01N 1/02, C12N 5/071

(54) **COMPOSITION FOR PRESERVATION OF CELLS, TISSUES OR ORGANS, COMPRISING ISOLATED MITOCHONDRIA, AND USE THEREOF**

(30) Priority: 23.07.2021 KR 20210097204
(71) Applicant: Paean Biotechnology Inc., Daejeon 34013 (KR)
(72) Inventor: KANG, Young-Cheol, Wonju-si Gangwon-do 26463 (KR); HAN, Kyuboem, Daejeon 34119 (KR); KIM, Chun-Hyung, Goyang-si Gyeonggi-do 10413 (KR); LEE, Hong Kyu, Seoul 04425 (KR); KIM, Soomin, Seoul 02754 (KR); TANOTO, Hendra, Seoul 03368 (KR); ZHANG, Yin Hua, Seoul 03070 (KR); LIN, Zhi Jun, Seoul 03080 (KR); CUI, Hui Xing, Seoul 03080 (KR)
(74) Representative: HGF
(86) International application number: PCT/KR2022/010765
(87) International publication number: WO 2023/003418

(57) **Abstract**

The present invention relates to a preservation solution composition for the preservation of an isolated cell, tissue, or organ, comprising isolated mitochondria; and a method for preserving an isolated cell, tissue, or organ by using the same. The preservation solution composition according to the present invention has excellent long-term preservability while maintaining the activity of isolated cell, tissue, or organ. Therefore, the present invention may be effectively used in the transplantation medicine field for ultimately improving transplant success rates and in the regenerative medicine field and the like.

## Description

### Technical Field

The present invention relates to: a preservation solution composition for the preservation of an isolated cell, tissue, or organ, comprising isolated mitochondria; and a method for preserving the isolated cell, tissue, or organ by using the same.

### Background Art

Organ transplantation refers to replacing a dysfunctional tissue or organ with another normal tissue or organ. With the development of immunosuppressants in the 1980s, the success rate of organ transplant surgery was increased, and excellent organ transplant surgery methods are being developed to this day. In addition, after the 'Organs Transplant Act' was implemented in Korea in 2020, the number of organ transplant surgeries through organ donation from brain-dead people was increased rapidly.

Because it is difficult for a tissue or organ isolated from a donor to maintain its function for a long time *in vitro,* the success rate of transplantation into living organisms can be increased if transplanted as soon as possible. However, unfortunately, there is a problem that it takes time for the isolated tissue or organ to be delivered to the patient in need of a transplant, which ultimately results in the tissue or organ becoming unusable or the function of the transplanted tissue or organ deteriorating. If a tissue or an organ can be preserved without damaging the function of the isolated tissue or organ, the tissue or organ can be transported much more easily and the patient can be prepared for transplantation, valuable tissues or organs will be not discarded, and the success rate of transplantation into living organisms can also be increased. Recently, research has been conducted to preserve tissues and organs (WO2021-007275A1).

However, the development of a preservation solution or device that can maintain the activity of isolated tissues or organs for a long time is still insufficient. Therefore, there is a need to develop a preservation solution or device that can maintain the biological function of the isolated tissues or organs and preserve the isolated tissues or organs for a long period of time.

### Detailed Description of Invention

### Technical Problem

Accordingly, the present inventors have tried to develop a preservation solution that can preserve an isolated cell, tissue, or organ for a long period of time. As a result, it was confirmed that the preservation solution comprising isolated mitochondria can maintain the activity of the isolated cell, tissue, or organ for a long period of time. Therefore, the present inventors developed a preservation solution that can be effectively used for the preservation of the cell, tissue, or organ.

### Solution to Problem

In order to achieve the above object, in one aspect of the present invention, there is provided a preservation solution composition for a preservation of an isolated cell, tissue, or organ, comprising isolated mitochondria.

In another aspect of the present invention, there is provided a method for preserving an isolated cell, tissue, or organ, comprising: storing the cell, tissue, or organ in the presence of the preservation solution composition.

### Effects of Invention

The preservation solution comprising isolated mitochondria according to the present invention may preserve an isolated cell, tissue, or organ for a long period of time while maintaining the activity of the isolated cell, tissue, or organ. Therefore, the activity of tissues of a living organism for transplantation may be maintained, and ultimately, the transplant success rate may be increased with a high possibility. Accordingly, it has high potential for industrial applicability not only in the transplantation medicine field but also in the regenerative medicine field.

### Brief Description of Drawings

Figure 1 is a graph showing normalized perfusate volume versus time (h) for which isolated hearts were stored in a mitochondria-containing histidine-tryptophan-ketoglutarate (HTK) solution or a mitochondria-free HTK solution.
Figure 2 shows images of myofibrillar cells according to storage time (h) when isolated hearts were stored in a mitochondria-HTK solution or a mitochondria-free HTK solution.
Figure 3 shows images of isolated organs after storage in a mitochondria-HTK solution or a mitochondria-free HTK solution.
Figure 4 is a graph showing the results of analyzing the level of ACE2 enzyme activity in the kidney when the isolated kidney was stored in a mitochondria-HTK solution or a mitochondria-free HTK solution.
Figure 5 is a confocal microscope photograph showing the results of confirming the movement of mitochondria after storing isolated cardiomyocytes in a HTK solution with or wihout a mitochondria, which is stained with MitoTracker^{™}. Here, hPl-mito indicates human platelet-derived mitochondria.
Figure 6 is a diagram showing the results of confirming the degree of increase in cardiomyocyte survival rate after storing isolated cardiomyocytes in a mitochondria-HTK solution or a mitochondria-free HTK solution, and shows the results of WST-1 assay measurement.
Figure 7 is a diagram showing the results of confirming the degree of increase in cardiomyocyte survival rate after storing isolated cardiomyocytes in a mitochondria-HTK solution or a mitochondria-free HTK solution, and shows the results of intracellular ATP measurement.
Figure 8 is a confocal microscope photograph showing the results of confirming the movement of mitochondria within the heart tissue after storing the isolated heart tissue in a mitochondria-HTK solution or a mitochondria-free HTK solution.
Figure 9 is a graph showing the results of analyzing the level of ATP synthase activity in the heart when the isolated heart was stored in a mitochondria-HTK solution or a mitochondria-free HTK solution.
Figure 10 is a graph showing the results of analyzing the level of citrate synthase activity in the heart when the isolated heart was stored in a mitochondria-HTK solution or a mitochondria-free HTK solution.
Figure 11 is a graph showing the results of analyzing the level of ACE2 enzyme activity in the kidney when the isolated kidney was stored in a mitochondria-containing mitochondria-HTK solution at various concentration.
Figure 12 is a graph showing the results of analyzing the level of ACE2 enzyme activity in the kidney when the isolated kidney was stored in activated or inactivated various origin-mitochondria-HTK solutions.

### Best Mode for Carrying out the Invention

### Preservation solution composition comprising mitochondria

In one aspect of the present invention, there is provided a preservation solution composition for a preservation of an isolated cell, tissue, or organ, comprising isolated mitochondria.

As used herein, the term "mitochondria" is a cellular organelle essential for the survival of eukaryotic cells that is involved in the synthesis and regulation of adenosine triphosphate (ATP), an energy source. The mitochondria are involved in various metabolic pathways *in vivo,* such as cell signaling, cell differentiation, cell death, as well as control of the cell cycle and cell growth.

As used herein, the term "isolated mitochondria" may refer to mitochondria obtained from autologous, allogeneic, or xenogeneic sources.

As used herein, the term "autologous mitochondria" refers to mitochondria obtained from tissues or cells of the same subject. In addition, the term "allogeneic mitochondria" refers to mitochondria obtained from tissues or cells of another subject belonging to the same species as the subject and having different genotypes with respect to alleles. In addition, the term "xenogeneic mitochondria" refers to mitochondria obtained from tissues or cells of a subject belonging to a different species.

In this case, the subject may be a mammal, and preferably may be a human.

The mitochondria may be isolated from tissues or cells of a subject. For example, the mitochondria may be obtained from somatic cells, germ cells, or stem cells, or may be isolated from blood cells or platelets. In addition, the mitochondria may be normal mitochondria obtained from cells with normal mitochondrial biological activity. In addition, the mitochondria may be obtained from autologous or allogeneic cells cultured *in vitro.*

As used herein, the term "cell" refers to a structural or functional unit constituting a living organism, consisting of cytoplasm surrounded by a cell membrane, and comprising biomolecules such as proteins and nucleic acids. The cell refers to a cell comprising mitochondria inside the cell membrane.

As used herein, the term "somatic cell" refers to cells that make up an organism, excluding germ cells. The somatic cells may be any one selected from the group consisting of muscle cells, hepatocytes, nerve cells, fibroblasts, epithelial cells, adipocytes, osteocytes, leukocytes, lymphocytes, platelets, mucosal cells, and a combination thereof. Preferably, it may be obtained from muscle cells or hepatocytes with excellent mitochondrial activity. In addition, it may be obtained from autologous or allogeneic blood PBMC cells.

As used herein, the term "platelet" refers to a type of the blood cell that is a formed element of blood. It is produced in the bone marrow and plays an important role in hemostasis and blood coagulation after blood vessels or tissues are damaged. The mitochondria may be obtained from autologous or allogeneic platelets.

As used herein, the term "germ cell" is a cell that participates in reproduction and includes sperm and eggs, which are gametes of organisms that reproduce sexually, and ancestral cells at all stages of their development. The mitochondria may be obtained from autologous or allogeneic germ cells. The germ cells may be sperm or eggs.

As used herein, the term "stem cell" refers to an undifferentiated cell that has the ability to differentiate into various types of tissue cells. The stem cells may be any one selected from the group consisting of mesenchymal stem cells, adult stem cells, dedifferentiated stem cells, embryonic stem cells, bone marrow stem cells, neural stem cells, limbal stem cells, tissue-derived stem cells, and a combination thereof.

In this case, the mesenchymal stem cell may be any one selected from the group consisting of umbilical cord, umbilical cord blood, bone marrow, fat, muscle, nerve, skin, amniotic membrane, placenta, synovial fluid, testis, periosteum, and a combination thereof. Preferably, it may be derived from human umbilical cord.

In addition, the mitochondria may be obtained by concentrating and disrupting cell or tissue samples isolated from a subject or cultured *in vitro.* In addition, the mitochondria may be obtained by disrupting cell or tissue samples that have been thawed after freeze storage.

In addition, the mitochondria may be obtained from cell or tissue samples that have been freeze-dried, freeze-stored, or thawed after freeze storage, but are not particularly limited as long as the biological activity of the obtained mitochondria is not negatively affected.

In addition, the mitochondria may be undamaged and have normal biological activity. Specifically, the mitochondria with normal biological activity may have one or more characteristics selected from the group consisting of (i) having a membrane potential, (ii) generating ATP within the mitochondria, and (iii) removing ROS or reducing the activity of ROS within the mitochondria.

The preservation solution composition may further comprise at least one selected from the group consisting of histidine, tryptophan, and ketoglutarate.

The ketoglutarate may be alpha-ketoglutarate.

The composition may comprise a mixture of isolated mitochondria and at least one selected from the group consisting of histidine, tryptophan, and ketoglutarate.

The concentration of isolated mitochondria in the composition may be from about 0.01 µg/mL to about 1 mg/mL, from about 0.01 µg/mL to about 100 µg/mL, from about 0.01 µg/mL to about 90 µg/mL, from about 0.01 µg/mL to about 80 µg/mL, from about 0.01 µg/mL to about 70 µg/mL, from about 0.01 µg/mL to about 60 µg/mL, from about 0.01 µg/mL to about 50 µg/mL, from about 0.01 µg/mL to about 40 µg/mL, from about 0.01 µg/mL to about 30 µg/mL, from about 0.01 µg/mL to about 20 µg/mL, from about 0.01 µg/mL to about 10 µg/mL, from about 0.01 µg/mL to about 9 µg/mL, from about 0.01 µg/mL to about 8 µg/mL, from about 0.01 µg/mL to about 7 µg/mL, from about 0.01 µg/mL to about 6 µg/mL, from about 0.01 µg/mL to about 5 µg/mL, from about 0.1 µg/mL to about 10 µg/mL, from about 0.5 µg/mL to about 10 µg/mL, from about 1 µg/mL to about 10 µg/mL, from about 2.5 µg/mL to about 10 µg/mL, or from about 5 µg/mL to about 10 µg/mL.

The concentration of histidine in the composition may be from about 1 mM to about 500 mM, from about 5 mM to about 500 mM, from about 10 mM to about 500 mM, from about 25 mM to about 500 mM, from about 50 mM to about 500 mM, from about 75 mM to about 500 mM, from about 100 mM to about 500 mM, from about 125 mM to about 500 mM, from about 150 mM to about 500 mM, from about 175 mM to about 500 mM, from about 198 mM to about 500 mM, from about 100 mM to about 400 mM, from about 100 mM to about 300 mM, from about 100 mM to about 200 mM, or from about 100 mM to about 198 mM.

The concentration of tryptophan in the composition may be from about 1 µM to about 100 mM, from about 1 µM to about 90 mM, from about 1 µM to about 80 mM, from about 1 µM to about 70 mM, from about 1 µM to about 60 mM, from about 1 µM to about 50 mM, from about 1 µM to about 40 mM, from about 1 µM to about 30 mM, from about 1 µM to about 20 mM, from about 1 µM to about 10 mM, from about 1 µM to about 5 mM, from about 10 µM to about 10 mM, from about 100 µM to about 10 mM, from about 0.5 mM to about 10 mM, from about 1 mM to about 10 mM, from about 1.5 mM to about 10 mM, or from about 2 mM to about 10 mM.

The concentration of ketoglutarate in the composition may be from about 1 µM to about 100 mM, from about 1 µM to about 90 mM, from about 1 µM to about 80 mM, from about 1 µM to about 70 mM, from about 1 µM to about 60 mM, from about 1 µM to about 50 mM, from about 1 µM to about 40 mM, from about 1 µM to about 30 mM, from about 1 µM to about 20 mM, from about 1 µM to about 10 mM, from about 1 µM to about 5 mM, from about 10 µM to about 10 mM, from about 100 µM to about 10 mM, from about 250 µM to about 10 mM, from about 500 µM to about 10 mM, from about 750 µM to about 10 mM, from about 1 mM to about 10 mM, from about 1 mM to about 5 mM, or from about 1 mM to about 2 mM.

In addition, the preservation solution composition may further comprise a cold storage solution or a cryopreservation solution for the long-term preservation of the isolated cell, tissue, or organ.

The cold storage solution may comprise hydroxyethyl starch (HES), N-acetyl cysteine (NAC), and dexamethasone (DEX). The HES may be tetra-hydroxyethyl starch (T-HES), penta-hydroxyethyl starch (P-HES), or hexa-hydroxyethyl starch (H-HES), but is not limited thereto.

The cold storage solution may further comprise essential amino acids, insulin, and glucose, but is not limited thereto.

The cryopreservation solution may comprise hydroxyethyl starch (HES) and dimethyl sulfoxide (DMSO). The cryopreservation solution may comprise a cell culture medium such as DMEM or MEM, and a physiological buffer such as PlasmaLyte or PBS.

Specifically, the cryopreservation solution may comprise at least one additional cryoprotectant substance selected from the group consisting of acetamide, agarose, alginate, 1-analine, albumin, ammonium acetate, butanediol, chondroitin sulfate, chloroform, choline, diethylene glycol, dimethyl acetamide, methyl formamide, dimethyl sulfoxide (DMSO), erythritol, ethanol, ethylene glycol, formamide, glucose, glycerol, α-glycerol phosphate, glycerol monoacetate, glycine, hydroxyethyl starch, inositol, lactose, magnesium chloride, magnesium sulfate, maltose, mannitol, mannose, methanol, methyl acetamide, methylformamide, methyl urea, phenol, pluronic polyol, polyethylene glycol, polyvinylpyrrolidone, proline, propylene glycol, pyridine N-oxide, ribose, serine, sodium bromide, sodium chloride, sodium iodide, sodium nitrate, sodium sulfate, sorbitol, sucrose, trehalose, triethylene glycol, trimethylamine acetate, urea, valine, xylose, dextrin, dextran, and isomaltooligosaccharide.

On the other hand, the preservation solution composition may further comprise IAP (inhibitors of apoptosis), rho-associated protein kinase (ROCK) signal pathway inhibitors, and/or proteins belonging to growth factors such as EGF, FGF, PDGF, IGF, EPO, BDNF, TGF, TNF, and/or VEGF.

In addition, the preservation solution composition may comprise one or more additives selected from antioxidants, osmotic regulating agents, and energy sources.

The antioxidants may include, but are not limited to, alpha tocopherol (vitamin E), alpha tocopherol acetate, ascorbic acid, ascorbyl palmitate, butylated hydroxyanisole butylated hydroxyquinone, butylated hydroxytoluene, hydroxycoumarin, ethyl gallate, propyl gallate, octyl gallate, lauryl gallate, propyl hydroxybenzoate, trihydroxybutyrophenone, dimethylphenol, lecithin, coenzyme Q10, ethanolamine, or a combination thereof.

The osmotic regulating agents may include, but are not limited to, dextran, sodium chloride, glucose, sorbitol, xylitol, mannitol, dextrose, sodium bicarbonate, calcium chloride, potassium chloride, sodium lactate, Ringer's solution, lactated Ringer's solution, hydroxyethyl starch, raffinose, glycerol, or a combination thereof.

The energy sources may include, but are not limited to, adenosine-5-triphosphate (ATP), adenosine-5-triphosphate disodium salt, glucose, maltose, mannose, galactose, or fructose, and may be adenosine-5-triphosphate (ATP) and/or adenosine-5-triphosphate disodium salt.

In one embodiment, the preservation solution composition may comprise adenosine, glutathione, BES (N,N-bis[2-hydroxyethyl]-2-aminoethanesulfonic acid), HES (hydroxyethyl starch), or PEG (polyethylene glycol) as an active ingredient, in addition to at least one substance selected from the group consisting of histidine, tryptophan, and ketoglutarate. In addition, EC (Euro-Collins) solution for cold storage, UW (University of Wisconsin) solution (Via-span^{®}), Celsior^{®}, Custodiol^{®}, IGL-1^{®}, BGP-HMP (N,N-bis-2-hydroxyethyl-2-aminoethanesulfonic acid (BES)-gluconate-polyethylene glycol [BGP]-based solution (hypothermic machine perfusion [HMP]) solution, BGP-CS (cold storage) solution, and the like may also be added, but are not limited thereto.

The cell, tissue, or organ may be derived from a mammal. The mammal may be a human, cow, horse, pig, dog, sheep, goat, or cat.

The cell, tissue, or organ may be for transplantation into living organisms.

The cells include cells constituting tissue or organ, examples of which may include, but are not limited to, muscle cells, hepatocytes, fibroblasts, epithelial cells, nerve cells, adipocytes, osteocytes, leukocytes, lymphocytes, stem cells, or mucosal cells.

The tissue may be selected from skin, muscle, cornea, cartilage, nerve, and bone.

The organ may be any one selected from heart, liver, kidney, small intestine, stomach, pancreas, lung, gallbladder, nerve, skin, blood vessel, tooth, eye, cornea, brain, spinal cord, thyroid, bone marrow, muscle, thymus, spleen, large intestine, testis, and ovary.

The preservation solution composition may further comprise an additive that may increase the long-term preservation efficiency of isolated cell, tissue, or organ. The additive may be water, sugars (e.g., sucrose, raffinose, starch), pH buffers (e.g., sodium phosphate, potassium phosphate), ATP, buffering agents, albumin, or antioxidants (e.g., vitamin E).

### Composition for perfusion or re-perfusion comprising mitochondria

In another aspect of the present invention, there is provided a composition for perfusion or re-perfusion comprising isolated mitochondria.

The perfusion may be the replacement of protoplasm with an artificial fluid by injecting or continuously flowing a perfusion fluid into the isolated cell, tissue, or organ. The re-perfusion may be a temporary cessation of blood supply to the isolated cell, tissue, or organ and then restoration of blood flow.

The composition may be a composition for parenteral administration. The composition may be a liquid. In addition, the composition may comprise one or more additives. The additives may include excipients, diluents, anti-adherents, preservatives, vehicles, surfactants, or a combination thereof. In addition, the composition may further comprise salts, such as MgCl₂, CaCl₂, NaCl, KCl, or a combination thereof.

The composition may be a pharmaceutical composition.

### Method for preserving a cell, tissue, or organ using preservation solution composition

In another aspect of the present invention, there is provided a method for preserving an isolated cell, tissue, or organ, comprising: storing the isolated cell, tissue, or organ in the presence of a preservation solution composition comprising isolated mitochondria.

The cell, tissue, organ, mitochondria, and preservative solution compositions are as described above.

As used herein, the term "in the presence of a preservation solution composition" may refer to bringing the preservation solution composition of the present invention into contact with an object to be preserved, that is, a cell, tissue, or organ.

In one embodiment, this is a method for preserving a cell, tissue, or organ, etc. by immersing the cell, tissue, or organ, etc. in the preservation solution composition of the present invention, thereby bringing the preservation solution into contact with an object to be preserved. The amount of a preservation solution to an object to be preserved is not particularly limited, but an amount greater than that in which the object to be preserved is completely immersed may be preferable.

The preservation container is not particularly limited, and either a sealed container or an open container may be used. It may be selected appropriately depending on the purpose. Preferably, it may be a container that may maintain aerobic conditions (conditions in which gases such as oxygen, carbon dioxide, and nitrogen may freely breathe).

The storage may be performed in refrigerated conditions. The refrigerated conditions may be from about -4°C to about 25°C, from about -4°C to about 20°C, from about -4°C to about 15°C, from about -4°C to about 10°C, from about -2°C to about 10°C, from about -2°C to about 8°C, from about 0°C to about 15°C, from about 0°C to about 10°C, from about 0°C to about 8°C, from about 0°C to about 6°C, from about 0°C to about 5°C, from about 0°C to about 4°C, from about 0°C to about 3°C, or from about 2°C to about 5°C.

On the other hand, when the object to be preserved is cryopreserved by immersing it in the preservation solution composition of the present invention, a known cryoprotectant (e.g., DMSO, glycerin, etc.) may be added to the preservation solution composition as desired.

The storage period may be set for an appropriate period depending on the purpose. Specifically, The storage may be performed for up to about 10 days, up to about 9 days, up to about 8 days, up to about 7 days, about 0 hour to about 7 days, about 0 hour to about 6 days, about 0 hour to about 5 days, about 0 hour to about 4 days, about 0 hour to about 3 days, about 0 hour to about 2 days, about 0 hour to about 42 hours, about 0 hour to about 36 hours, about 0 hour to about 30 hours, about 0 hour to about 24 hours, about 0 hour to about 18 hours, about 0 hour to about 12 hours, about 0 hour to about 9 hours, about 0 hour to about 6 hours, or about 0 hour to about 3 hours, but is not limited thereto.

The method may further comprise the isolating mitochondria from cells. Methods known in the art may be used in the isolating mitochondria from cells. The isolating mitochondria from cells may comprise lysing the cells and isolating mitochondria from the lysate. The isolation may be performed by centrifugation.

### Mode for Carrying out the Invention

Hereinafter, the present invention will be described in more detail by way of the following examples. However, the following examples are only for illustrating the present invention, and the scope of the present invention is not limited thereto.

### Example 1. Confirmation of function of heart according to application of isolated mitochondria-containing solution

### Example 1.1. Preparation of platelets

About 40 mL to about 50 mL of concentrated human platelets were centrifuged at 2,000xg for about 30 minutes. The supernatant containing plasma was removed, and the resulting platelet precipitate (pellet) was obtained. The obtained platelet precipitate was resuspended in the same volume of TTG mixed preservation solution (0.195 M trehalose, 20 mM Tris, and 65 mM glycine: TTG) as plasma. The resuspended platelet precipitate was dispensed in 1 mL portions and stored at -80°C until use.

### Example 1.2. Obtaining mitochondria from platelets

The frozen stored platelets were thawed and disrupted by vortexing for about 5 minutes, and centrifuged for about 5 minutes at 2,000xg. The resulting supernatant was centrifuged at 12,000xg for 10 minutes to obtain precipitated mitochondria.

### Example 1.3. Stabilization of isolated heart

In order to confirm the function of the heart, the isolated heart was first stabilized.

Specifically, SD (Sprague-Dawley) rats (12-week-old, male) were anesthetized with ketamine and then sacrificed. The heart of the rat was extracted, and the isolated heart was placed in Langendorff via an aortic catheter. The isolated heart was perfused with a Krebs-Henseleit solution for about 20 minutes to wash the heart. A refrigerated stored histidine-tryptophan-ketoglutarate (HTK) solution was injected through the aorta of the heart, and perfused for about 10 minutes to stabilize the heart.

The HTK solution contained 198 mM histidine, 2 mM tryptophan, 1 mM ketoglutarate, 30 mM mannitol, 4 mM MgCl₂, 15 µM CaCl₂, 15 mM NaCl, and 9 mM KCl.

### Example 2. Confirmation of perfusate maintenance in extracted heart according to application of mitochondria-containing solution

The mitochondria prepared in Example 1.2. were added to the HTK solution to prepare a mitochondria-HTK solution comprising 5 µg/mL of mitochondria.

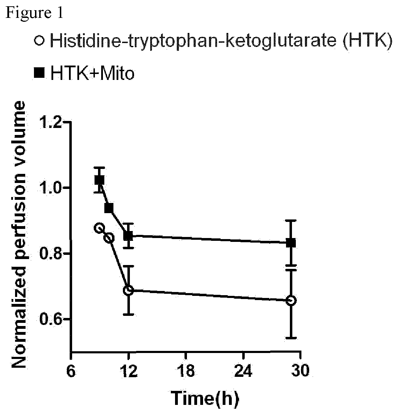

The stabilized heart prepared in Example 1.3. was injected with a mitochondria-HTK solution comprising 5 µg/mL of mitochondria or a mitochondria-free HTK solution for about 2 minutes. Thereafter, the heart was immersed in 150 µg/30 mL of a mitochondria-HTK solution and 30 mL of a mitochondria-free HTK solution, respectively, and stored at about 4°C. After 10, 12, and 29 hours, the stored heart was placed in Langendorff via an aortic catheter, and a Krebs-Henseleit solution was passed through it. The amount of perfusate flowing through the heart was measured. At this time, the change in the amount of perfusate is heart rate-dependent and is related to the heart rate. Therefore, the normalized perfusate volume (mL) over time was measured and shown in Figure 1.

As shown in Figure 1, the perfusate of the heart was increased by about 20% when stored in the mitochondria-HTK solution compared to when stored only in the mitochondria-free HTK solution at 9, 10, 12, and 29 hours.

In addition, after isolating the heart, the isolated heart was stored in a mitochondria-HTK solution or a mitochondria-free HTK solution stored, and then the duration of heartbeat was checked.

At 9 hours after heart isolation, the beats of hearts stored in a mitochondria-HTK solution or a mitochondria-free HTK solution were clearly distinguishable. The heart stored in the mitochondria-free HTK solution had a very weak heartbeat after 9 hours, but the heart stored in the mitochondria-HTK solution had a faster and more consistent heartbeat than the heart stored in the mitochondria-free HTK solution even after 9 hours.

Therefore, it was confirmed that the isolated heart maintained cardiac contractility for a long period of time in the presence of a mitochondria-containing solution.

### Example 3. Confirmation of maintenance of myofibrillar cells in extracted heart according to application of mitochondria-containing solution

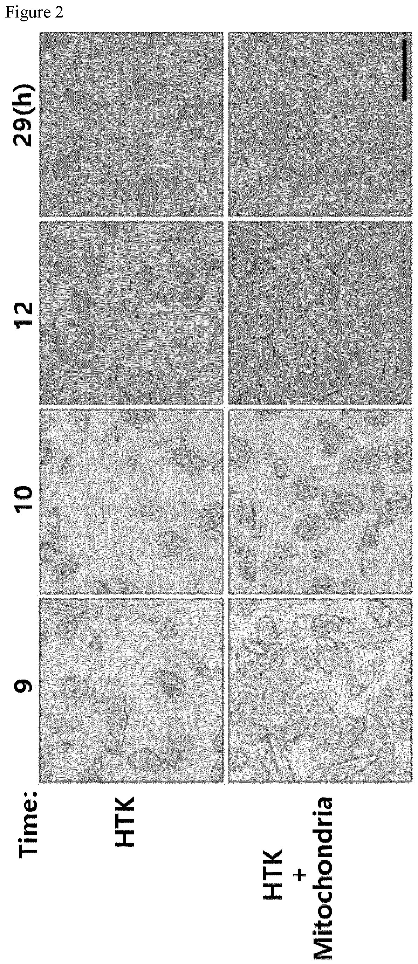

Myofibrillar cells were isolated from the heart whose perfusate was measured in Example 2. The condition of the isolated cells was observed under a microscope. When the isolated heart was stored in a mitochondria-HTK solution or a mitochondria-free HTK solution, images of myofibrillar cells according to storage time (9, 10, 12, and 29 hours) are shown in Figure 2.

As shown in Figure 2, when stored in a mitochondria-HTK solution, cardiomyocytes of the heart were isolated about 2 to 3 times more than cells stored in a mitochondria-free HTK solution.

Therefore, it was confirmed that when the heart was stored in the presence of a mitochondria-containing solution, the number of cardiomyocytes in the heart was increased compared to the case without mitochondria.

### Example 4. Confirmation of function of heart, liver, and kidney stored in solution containing isolated mitochondria

### Example 4.1. Preparation for confirmation of function of isolated heart, liver, and kidney

C57BL6 mice (7-week-old, male) were anesthetized with ketamine, and then the heart, liver and kidney were extracted. The extracted heart, liver, and kidney were washed several times with PBS to remove blood and foreign substances. The washed organs were immersed in a refrigerated stored mitochondria-free HTK solution and a mitochondria-HTK solution, respectively, and then refrigerated stored.

### Example 4.2. Confirmation of chromaticity change in extracted heart, liver, and kidney according to application of mitochondria-containing solution

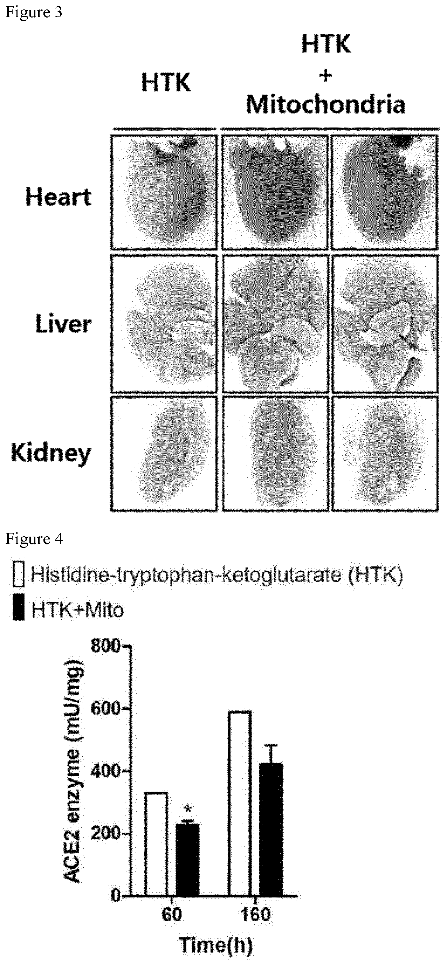

The heart, liver, and kidney stored in Example 4.1. were washed several times with PBS and then photographed to compare the chromaticity. As shown in Figure 3, the heart maintained its bright red color when stored in a mitochondria-HTK solution, and the red color disappeared when stored in a mitochondria-free HTK solution. Similar results were seen in the liver and kidney.

### Example 4.3. Confirmation of maintenance of enzyme activity in extracted kidney according to application of mitochondria-containing solution

The entire kidney stored in Example 4.1. was homogenized 60 and 160 hours later, respectively, and then the activity of ACE2 (angiotensin II converting enzyme), which is highly expressed in the kidney, was measured according to the manufacturer's manual using the ACE2 Activity Assay Kit (Catalog # K897-100, Biovision). As shown in Figure 4, the activity of ACE2 was increased with time in the mitochondria-free HTK solution and the mitochondria-HTK solution. In particular, it was confirmed that when the kidney was stored in a mitochondria-HTK solution, the activity of ACE2 was increased more slowly than when stored only in a mitochondria-free HTK solution, that is, the increase in ACE2 activity was delayed.

In other words, the expression and activity of ACE2 is increased due to the continuous decrease in energy and increase in stress after organ isolation. As a result, it was found that the increased activity of ACE2 reduces stress in the organ and provides energy according to the treatment with mitochondria, thereby reducing the activity of ACE2.

### Example 5. Confirmation of effect of application of mitochondria-containing solution to extracted heart

### Example 5.1. Isolation of cardiomyocytes from extracted heart

The left ventricle fragment of the extracted heart was treated with collagenase (Worthington Biochemical Co. Cat. No. LS004194) and protease (Sigma Aldrich, Cat. No. p4630) for 5 minutes, and then centrifuged at 2,000xg to obtain cardiomyocytes.

### Example 5.2. Staining of isolated mitochondria

The mitochondria isolated from platelets were stained with MitoTracker^{™} (ThermoFisher, Cat. No. M7512), a mitochondria-specific staining reagent, for 10 minutes. Thereafter, the supernatant was removed by centrifugation at 12,000xg and resuspended in a mitochondria isolation solution.

### Example 5.3. Confirmation of increase in cardiomyocyte survival rate according to application of mitochondria-containing solution

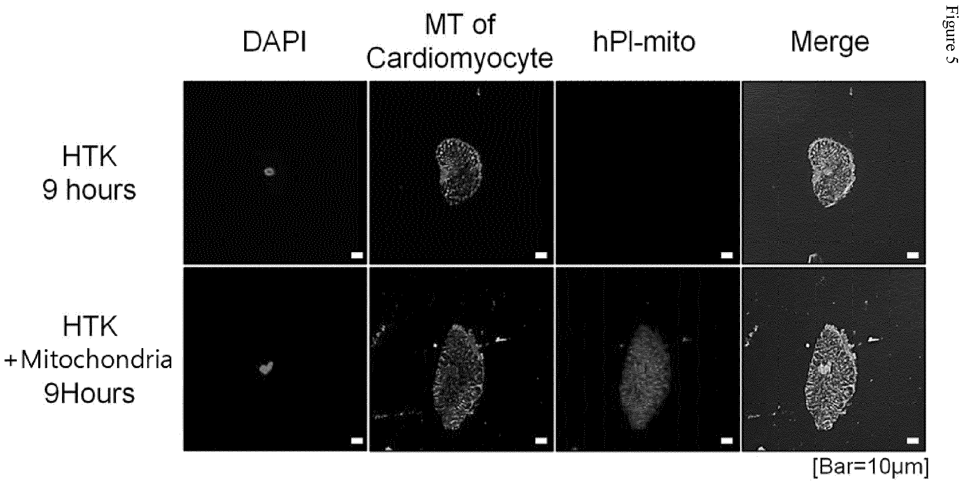

The cardiomyocytes isolated in Example 5.1. were stored in the mitochondria-free HTK solution and the stained mitochondria-HTK solution that had been prepared in Example 5.2. for 9 hours, and then the movement of mitochondria was confirmed using a confocal microscope. As a result, it was confirmed that the mitochondria in the solution moved into cardiomyocytes, as shown in Figure 5.

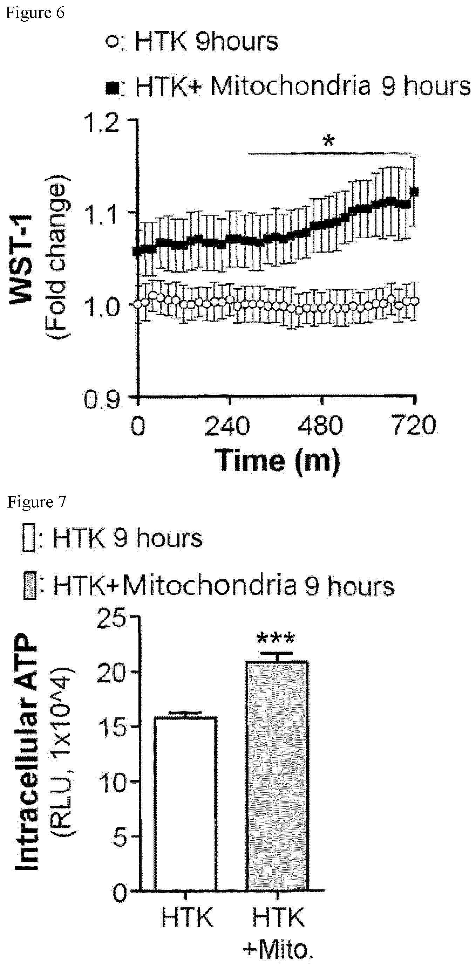

In addition, the increase in survival rate due to mitochondria moving into cardiomyocytes was confirmed in the results of measuring WST-1 (water-soluble tetrazolium salt) and intracellular ATP (Figures 6 and 7).

### Example 5.4. Confirmation of movement of mitochondria within heart tissue according to application of mitochondria-containing solution

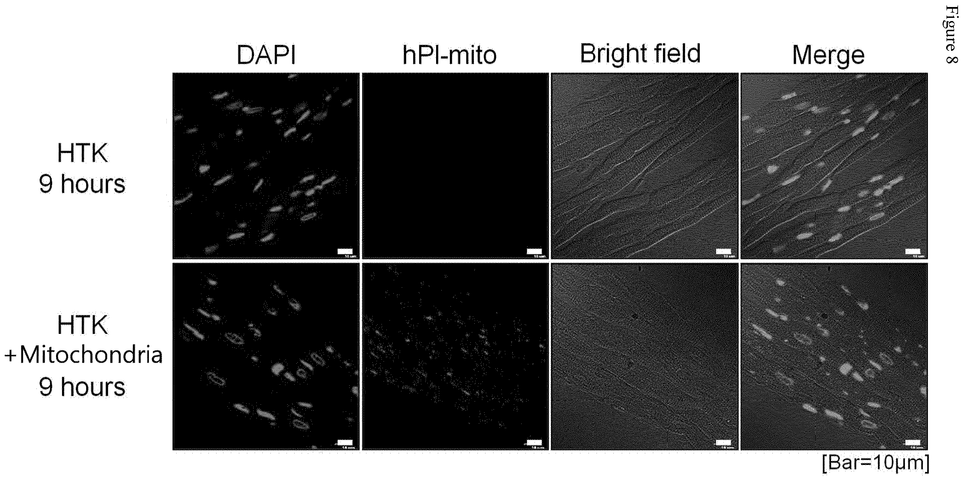

The extracted heart was stored in the mitochondria-free HTK solution and the stained mitochondria-HTK solution that had been prepared in Example 5.2. After 9 hours, it was fixed and sectioned. Thereafter, mitochondria that had moved into the tissue were confirmed using a confocal microscope (Figure 8).

### Example 5.5. Confirmation of increased activity of mitochondrial enzymes in heart tissue according to application of mitochondria-containing solution

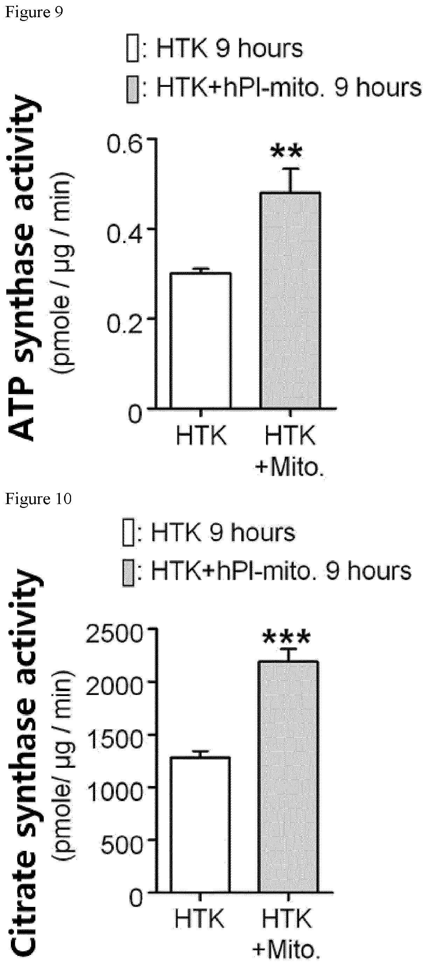

The mitochondria were isolated from the extracted heart tissue being stored, and then the activities of ATP synthase and citrate synthase were measured, respectively. As a result, as shown in Figures 9 and 10, when the mitochondria-HTK solution was applied, both ATP synthase and citrate synthase exhibited significantly higher activities.

### Example 6. Confirmation of maintenance of enzyme activity in kidney depending on mitochondria concentration

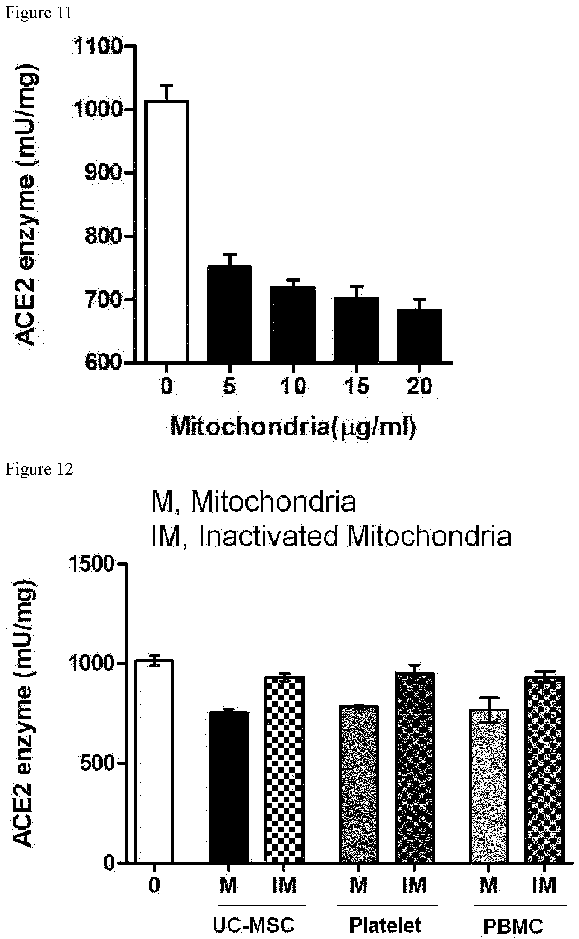

The kidney was prepared in the same manner as in Example 4.1., except for the storage time of 192 hours and the concentration-dependent treatment of a mitochondria-HTK solution (0, 5, 10, 15, and 20 µg/mL), and the activity of ACE2 was measured. As shown in Figure 11, it was confirmed that the activity of ACE2 was decreased by mitochondria in a concentration-dependent manner.

### Example 7. Confirmation of maintenance of enzyme activity according to application of mitochondria-containing solution depending on mitochondria origin and activation

The kidney was prepared in the same manner as in Example 4.1., except for the storage time of 192 hours and the treatment with a mitochondria-HTK solution with different mitochondria origin and activation, and the activity of ACE2 was measured. At this time, the inactivated mitochondria were treated by heating at 56°C for 30 minutes after sonication.

As a result, as shown in Figure 12, it was confirmed that the activity of ACE2 was not reduced when applying each heat-inactivated mitochondria, but the activity of ACE2 was reduced by mitochondria of various origins isolated from umbilical cord blood mesenchymal stem cells (UC-MSC), platelets, and peripheral blood mononuclear cells (PBMC).

## Claims

1. A preservation solution composition for the preservation of an isolated cell, tissue, or organ, comprising isolated mitochondria.

2. The preservation solution composition according to claim 1, wherein the mitochondria are obtained from platelets, muscle cells, hepatocytes, fibroblasts, epithelial cells, nerve cells, adipocytes, osteocytes, leukocytes, lymphocytes, stem cells, or mucosal cells.

3. The preservation solution composition according to claim 1, wherein the composition further comprises at least one selected from the group consisting of histidine, tryptophan, and ketoglutarate.

4. The preservation solution composition according to claim 1, wherein the composition comprises a mixture of isolated mitochondria and at least one selected from the group consisting of histidine, tryptophan, and ketoglutarate.

5. The preservation solution composition according to claim 1, wherein the concentration of isolated mitochondria in the composition is 0.01 µg/mL to 1 mg/mL.

6. The preservation solution composition according to claim 3 or 4, wherein the concentration of histidine in the composition is 1 mM to 500 mM.

7. The preservation solution composition according to claim 3 or 4, wherein the concentration of tryptophan in the composition is 1 µM to 100 mM.

8. The preservation solution composition according to claim 3 or 4, wherein the concentration of ketoglutarate in the composition is 1 µM to 100 mM.

9. The preservation solution composition according to claim 1, wherein the cell, tissue, or organ are a cell, tissue, or organ derived from a mammal.

10. The preservation solution composition according to claim 9, wherein the mammal is a human.

11. The preservation solution composition according to claim 1, wherein the cell, tissue, or organ are for transplantation into living organisms.

12. The preservation solution composition according to claim 1, wherein the organ is any one selected from heart, liver, kidney, small intestine, stomach, pancreas, lung, gallbladder, nerve, skin, blood vessel, tooth, eye, and cornea.

13. The preservation solution composition according to claim 1, wherein the composition is for perfusion or re-perfusion.

14. A method for preserving an isolated cell, tissue, or organ, comprising:
storing the isolated cell, tissue, or organ in the presence of a preservation solution composition comprising isolated mitochondria.

15. The method according to claim 14, wherein the storage is performed in refrigerated conditions.

16. The method according to claim 14, wherein the storage is performed for up to 10 days.
